# EUROPÄISCHE PATENTSCHRIFT

(11) **EP 1 123 354 B1**
(45) Veröffentlichungstag und Bekanntmachung des Hinweises auf die Patenterteilung: **07.01.2004**
(21) Anmeldenummer: 99950681.9
(22) Anmeldetag: 08.10.1999
(51) Int. Cl.: C09C 3/08, C08K 9/04

(54) **HOCHTEMPERATURBESTÄNDIGE POLYMERISIERBARE METALLOXIDPARTIKEL**
HIGH-TEMPERATURE RESISTANT POLYMERIZABLE METAL OXIDE PARTICLES
PARTICULES D'OXYDE METALLIQUE POLYMERISABLES RESISTANT AUX HAUTES TEMPERATURES

(30) Priorität: 09.10.1998 DE 19846660
(43) Veröffentlichungstag der Anmeldung: 16.08.2001
(73) Patentinhaber: Institut für Oberflächenmodifizierung e.V., 04303 Leipzig (DE); Hinterwaldner, Rudolf, 85614 Kirchseeon (DE); Hinterwaldner, Stephan, 85614 Kirchseeon (DE)
(72) Erfinder: HINTERWALDNER, Rudolf, 85614 Kirchseeon (DE); GLÄSEL, Hans-Jürgen, D-04229 Leipzig (DE); HARTMANN, Eberhard, D-04509 Priester (DE); MEHNERT, Reiner, D-04416 Markkleeburg (DE)
(74) Vertreter: Kinzebach, Werner, Dr.
(86) Internationale Anmeldenummer: PCT/EP1999/007569
(87) Internationale Veröffentlichungsnummer: WO 2000/022052

(56) Entgegenhaltungen:
- EP-A- 0 688 834
- WO-A-93/25611
- WO-A-94/07945
- DE-A- 4 233 396
- Encyclopedia of Polymer Science and Engineering, Band 4, "Coupling Agents", Seiten, 284-298
- E.P. Plueddemann, Silane Coupling Agents, Plenum Press, New York, 1982, p. 132
- A. Vidal and J.B. Donnet, Bull. Soc. Chim. Fr., 1985, 6, p.1088
- Manuskript über ein Vortrag von A. Tauber der auf dem RadTech-Symposium 2002, 28.04.-01.05.2002 in Indianapolis, USA, gehalten wurde

## Beschreibung

Die vorliegende Erfindung betrifft hochtemperaturbeständige polymerisierbare Metalloxidpartikel, Verfahren zu ihrer Herstellung, Zusammensetzungen welche diese Partikel enthalten und ihre Verwendung.

Nanoskalige, anorganische Materialien (Materialien mit einer mittleren Teilchengröße im Nanometerbereich), die mit organischen Resten oberflächenmodifiziert sind, sind bereits bekannt, siehe den unten noch näher diskutierten Stand der Technik. Die bekannten nanoskaligen Materialien einschließlich der Herstellungsverfahren haben eine Reihe von Nachteilen, weshalb ihre Verwendung fast ausschließlich auf das Aufbringen von harten Schichten auf Substratoberflächen begrenzt ist. Diese Nachteile sind überwiegend in der Herstellung der Materialien nach dem Sol-Gel-Prozeß begründet. Der Sol-Gel-Prozeß ist beispielsweise beschrieben in C. J. Brinker und G. Scherer "Sol-Gel-Science - The Physics and Chemistry of Sol-Gel-Processing", Academic Press, New York (1989) sowie in DE 1941191 A, DE 3719339 A und DE 4020316 A. Beim Sol-Gel-Prozeß werden anorganische Partikel, beispielsweise wäßrige kolloidale Siliziumdioxidlösungen (Wasserglas), mit Alkoxysilanen über Hydrolyse und Kondensationsreaktionen umgesetzt, wobei Gele unterschiedlicher oder sogar divergierender Eigenschaften erhalten werden.

Die Eigenschaften der nach dem Sol-Gel-Prozeß erhaltenen Partikel lassen sich durch Modifizierung der Oberfläche verändern. So wurde bereits die Umsetzung von kolloidalem Siliziumdioxid nach dem Sol-Gel-Prozeß mit acrylierten Alkoxysilanen in einem inerten organischen Lösungsmittel und die Anwendung der erhaltenen Produkte zur Herstellung von kratzfesten Beschichtungen beschrieben, siehe beispielsweise US 4,455,205, US 4,478,876 und Proceedings RadTech. North America '92, Seiten 457-461 (1992). In analoger Weise wurde bei der Einführung funktioneller Gruppen in strahlungshärtbare Sol-Gel-Beschichtungen verfahren, siehe New J. Chem. 18, 1117-1123 (1994) und DE 4338361 A. Weiter ist in Chem. Mater. 9, 1562-1569 (1967) die Modifizierung von kolloidalem Siliziumdioxid mit einem Trialkoxisilan, das Epoxy- oder 1-Propenylethergruppen aufweist, in wasserfreier, flüssiger organischer Phase beschrieben.

Die nach dem Sol-Gel-Prozeß hergestellten Partikel besitzen folgende Nachteile:
- Sie weisen keine reproduzierbaren Strukturen und Eigenschaften auf.
- Ihre Herstellung ist kostenintensiv und nicht immer umweltfreundlich.
- Die Lagerstabilitäten sind nicht zufriedenstellend.
- Sie sind nicht oder nur eingeschränkt mit anderen Monomeren copolymerisierbar.
- Die Menge an Partikeln, die in Substrate, wie Lacke etc. aufgenommen werden kann, ist begrenzt.

Der Einsatz der nach dem Sol-Gel-Prozeß erhaltenen Partikel beschränkt sich in der Praxis daher auf die Herstellung von kratzfesten, harten Beschichtungen.

Der vorliegenden Erfindung liegt nun die Aufgabe zugrunde, Partikel und Verfahren zu ihrer Herstellung zur Verfügung zu stellen, welche zumindest einen der genannten Nachteile nicht aufweisen. Insbesondere sollen Partikel zur Verfügung gestellt werden, die einfacher und wirtschaftlicher herstellbar sind und die auch für die Hochtemperaturanwendung geeignet sind.

Überraschenderweise wurde nun gefunden, daß diese Aufgabe gelöst wird, wenn man die Partikel nach einem Verfahren hestellt, bei dem man nicht von einem Sol ausgeht, sondern die Partikel als Feststoffe einsetzt und durch Umsetzung mit geeigneten Reagenzien die Oberfläche modifiziert und kovalent bindet.

Gegenstand der vorliegenden Erfindung sind daher hochtemperaturbeständige polymerisierbare Metalloxidpartikel mit einer Glasübergangstemperatur der Homopolymerisate von ≥ 100° C und mit einem Kern A aus mindestens einem Oxid eines Metalls oder Halbmetalls der dritten bis sechsten Hauptgruppe, der ersten bis achten Nebengruppe des Periodensystems oder der Lanthaniden und mit mindestens einer Gruppe -(B)w-X, die über ein oder mehrere Sauerstoffatome des Oxids oder Hydroxids kovalent an den Kern gebunden ist, wobei w für 0 oder 1 steht und B für einen Rest der Formeln

- (MeO)x Me(O)_{y1} ∼(R)_{y2}-

oder

-R(O)z-

steht, worin x für O bis 100, y1, y2 und z unabhängig voneinander für 0 oder 1 stehen und Me ein Metall oder Halbmetall der dritten bis sechsten Hauptgruppe oder der dritten bis achten Nebengruppe des Periodensystes bedeutet, wobei die freien Valenzen von Me eine Bindung an ein weiteres Sauerstoffatom des Kerns A und/oder eine Bindung über ein Sauerstoffatom an ein Me in einer anderen Gruppe B und/oder eine Bindung an ein Sauerstoffatom eines anderen Kerns A darstellen und/oder durch H, einen organischen Rest und/oder einen Trialkylsilyloxyrest abgesättigt sind;

R für divalentes Alkyl, Cycloalkyl, Aryl, Arylalkyl, Alkylaryl, Alkoxy, Acyl, Acyloxy oder für einen Rest steht, der nach Entfernung von zwei phenolischen Wasserstoffatomen aus einer mindestens zwei phenolische Hydroxylgruppen aufweisenden Phenolverbindung verbleibt, wobei R gegebenenfalls durch 1, 2 oder 3 Reste substituiert sein kann, die unabhängig voneinander ausgewählt sind unter Hydroxy, Alkoxy, Halogen und, im Falle von Aryl- oder Cycloalkylresten, auch Alkyl, und/oder durch ein oder zwei Sauerstoffatome in der Kette unterbrochen sein kann, und

X eine reaktionsfähige funktionelle Gruppe oder einen Rest mit einer reaktionsfähigen, funktionellen Gruppe bedeutet.

Weiter betrifft die Erfindung ein Verfahren zur Herstellung der hochtemperaturbeständigen, polymerisierbaren Metalloxidpartikel, wobei man die Reste B, B-X oder X an den in fester Form vorliegenden Kern A in Gegenwart einer starken Säure kovalent anbindet.

Im Rahmen der vorliegenden Erfindung gilt folgendes:

Alkyl (auch in Alkoxy, Alkylaryl etc.) bedeutet eine geradkettige oder verzweigte Alkylgruppe, die vorzugsweise 1 bis 50 Kohlenstoffatome, besonders bevorzugt 1 bis 20 Kohlenstoffatome, insbesondere 1 bis 12 Kohlenstoffatome und ganz besonders bevorzugt 1 bis 8 Kohlenstoffatome aufweist. Beispiele für Alkylgruppen sind Methyl, Ethyl, n-Propyl, i-Propyl, n-Butyl, i-Butyl, sec-Butyl, t-Butyl, n-Hexyl, n-Dodecyl und Stearyl.

Cycloalkyl steht vorzugweise für C₃-C₈-Cycloalkyl, insbesondere C₅-C₇-Cycloalkyl. Beispiele für Cycloalkylgruppen sind Cyclopropyl, Cyclobutyl, Cyclopentyl, Cyclohexyl und Cycloheptyl, wobei Cyclopentyl und Cyclohexyl bevorzugt sind.

Aryl (auch in Arylalkyl oder Alkylaryl) steht vorzugsweise für Phenyl oder Naphthyl.

Bevorzugte Arylalkylgruppen sind Benzyl oder Phenethyl.

Bevorzugte Alkylarylgruppen sind o-, m- oder p-Tolyl oder - Xylyl.

Beispiele für Alkoxygruppen sind Methoxy, Ethoxy, n-Propoxy, i-Propoxy, n-Butoxy, t-Butoxy etc.

Acyl bedeutet eine geradkettige oder verzweigte Alkyl- oder Arylcarbonylgruppe, die vorzugsweise 1 bis 50 Kohlenstoffatome oder 1 bis 18 Kohlenstoffatome, insbesondere 1 bis 12 Kohlenstoffatome und besonders bevorzugt 1 bis 6 Kohlenstoffatome aufweist. Bespiele für Acylgruppen sind Formyl, Acetyl, Propionyl, Butyryl, Benzoyl etc.. Entsprechendes gilt für Acyloxy. Beispiele für Acyloxy sind insbesondere Acetyloxy, Propionyloxy und Benzoyloxy.

Bei den divalenten Resten R befinden sich beide Bindungsstellen an beliebiger Stelle in dem Alkyl-, Cycloalkyl-, Aryl- oder Acylrest. Im Falle von divalentem Arylalkyl und Alkylaryl befindet sich eine Bindungsstelle im Arylteil und die andere im Alkylteil. Bei divalentem Acyl und Acyloxy befindet sich eine Bindungsstelle im Alkyl- oder Arylteil und die andere am Carbonylkohlenstoffatom bzw. am Sauerstoffatom. Divalentes Acyl und Acyloxy ist vorzugsweise so in die Gruppe B eingebunden, dass der Alkyl- oder Arylrest mit Me bzw. dem Sauerstoffatom von Me(O)_{y2} verbunden ist.

Die erfindungsgemäßen Partikel zeichnen sich dadurch aus, daß sie eine hohe Zahl von Seitenketten -(B)_{w}-X aufweisen. Die Zahl der Seitenketten ist größer als 2 und im allgemeinen liegt sie im Bereich von 10 bis 100, vorzugsweise 10 bis 50 und insbesondere 20 bis 50. Die Menge an Seitenketten -(B)_{w}-X, bezogen auf das Gesamtgewicht der Partikel, beträgt mindestens 10 Gew. -%, vorzugsweise mindestens 20 Gew.-% und besonders bevorzugt mindestens 50 Gew.-%. Die Seitenketten können bis zu 90 Gew.-%, vorzugsweise bis zu 75 Gew.-% der Partikel ausmachen.

Die mittlere Teilchengröße (bestimmt mittels Rasterelektronenmikroskopie) der erfindungsgemäßen Partikel kann bis zu 1 mm betragen. Im allgemeinen liegt sie im Bereich von 1 nm bis 0,5 mm, mit Vorteil im Bereich von 1 bis 500 nm, vorzugsweise 1 bis 300 nm bzw. 1 bis 100 nm, insbesondere 10 bis 50 nm. Die spezifische Oberfläche (BET, bestimmt nach DIN 66131) liegt im allgemeinen im Bereich von 50 bis 400 m²/g, vorzugweise 70 bis 300 m²/g.

Die Glasübergangstemperatur der Homopolymerisate der erfindungsgemäßen Partikel ist ≥ 100 °C, mit Vorteil ≥ 150 °C, vorzugsweise ≥ 250 °C, insbesondere ≥ 350 °C und besonders bevorzugt ≥ 400 °C. Die Obergrenze liegt im allgemeinen bei 600 °C, vorzugsweise bei 500 °C.

Der Kern A der erfindungsgemäßen Partikel wird aus einem Oxid mindestens eines Metalls oder Halbmetalls der dritten bis sechsten Hauptgruppe, der ersten bis achten Nebengruppe des Periodensystems oder der Lanthaniden gebildet. Der Ausdruck Oxid umfaßt auch Hydroxide und (gemischte) Oxid-Hydroxide. Es können Gemische unterschiedlicher Oxide oder Mischoxide zur Anwendung kommen. Der Kern weist an der Oberfläche Hydroxygruppen auf, über welche die Anbindung der Seitenketten -(B)_{w}-X erfolgt. Beim Kern A handelt es sich ebenfalls um Partikel, deren Teilchengrößen und Oberflächen in den oben genannten für die erfindungsgemäßen Partikel angegebenen Bereichen liegen.

Für den Kern A kommen vorzugsweise die Oxide folgender Metalle oder Halbmetalle in Frage:
Dritte Hauptgruppe: B, Al, Ga;
Vierte Hauptgruppe: Si, Ge und Sn;
Fünfte Hauptgruppe: As, Sb und Bi;
Sechste Hauptgruppe: Te;
Erste Nebengruppe: Cu;
Zweite Nebengruppe: Zn, Cd;
Dritte Nebengruppe: Sc, Y, La;
Vierte Nebengruppe: Ti, Zr, Hf;
Fünfte Nebengruppe: V, Nb;
Sechste Nebengruppe: Cr, Mo, W;
Siebte Nebengruppe: Mn;
Achte Nebengruppe: Fe, Co, Ni;
Lanthaniden: Ce, Yb, Lu.

Bevorzugt sind die Oxide von Metallen oder Halbmetallen der dritten und vierten Hauptgruppe sowie der ersten, vierten, sechsten und achten Nebengruppe des Periodensystems, sowie Gemische und Mischoxide davon.

Besonders bevorzugt sind die Oxide von Si, Al, Ti, Zr, sowie Gemische und Mischoxide davon.

Die Seitenketten der erfindungsgemäßen Partikel werden gebildet durch die funktionelle Gruppe X (wenn w = 0) oder durch Reste der Formeln:

- (MeO) ₓ Me(O)_{y1} -(R)_{y2}-X

oder

-R(O)_{z}-X

worin x, y1, y2 und z sowie Me die oben genannten Bedeutungen besitzen. Die Metalle bzw. Halbmetalle Me können gleich oder verschieden sein. Vorzugsweise ist Me eines der oben für den Kern A als bevorzugt genannten Metalle oder Halbmetalle. x steht vorzugsweise für 0 bis 10, insbesondere 0, 1, 2, 3 oder 4 und besonders bevorzugt für 0, 1 oder 2.

B ist vorzugsweise ausgewählt unter folgenden Resten:
a) -Me (O)_{y1}-R-
b) -Me-O-Me(O)_{y1}-R-
c) -Me-O-Me-O-Me(O)_{y1}-R-
d) -R-O-
e) -R-
worin y1 für 0 oder 1 steht und R die oben angegebenen Bedeutungen besitzt.

Weiterhin ist -Me-O-Me- im Rest b) vorzugsweise ausgewählt unter:

- Si-O-Si-

- Si-O-Al-

- Si-O-Ti-

- Si-O-Zr-

- Al-O-Ti-

- Al-O-Zr-

- Al-O-Al-

und -Me-O-Me-O-Me- im Rest c) steht vorzugsweise für -Si-O-Ti-O-Zr-.

Die Reihenfolge unterschiedlicher Metallatome im Rest - (MeO)ₓMe(O)_{y1}-(R)_{y2}- ist beliebig. Die Anbindung an den Kern kann über das eine oder das andere Metallatom erfolgen, z.B. -Si-O-Alkann so in die Seitenkette eingebaut sein, daß die Anbindung an den Kern entweder über das Si-Atom oder über das Al-Atom erfolgt.

Die Metalle bzw. Halbmetalle Me können über ein oder mehrere Sauerstoffatome des Kerns gebunden sein. Dies läßt sich anhand des folgenden Beispiels mit Me = Si veranschaulichen: 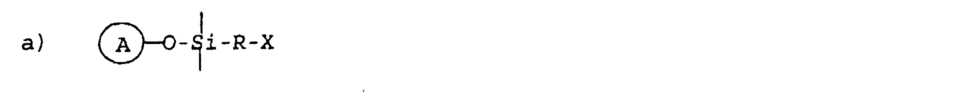 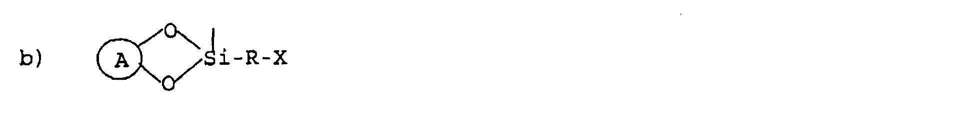 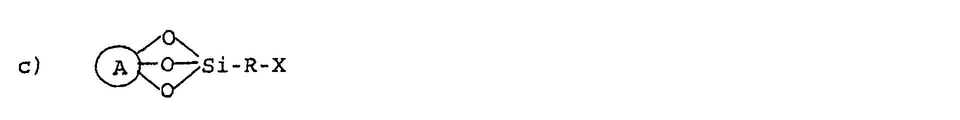 Wenn bei den Strukturen a) und b) die freien Valenzen des Si-Atoms durch Alkoxygruppen abgesättigt sind, kann durch AlkOH-Abspaltung eine Bindung an ein oder zwei weitere Kerne A erfolgen.

Die freien Valenzen von Me können auch eine Bindung über ein Sauerstoffatom an ein Me in einer anderen Gruppe B des gleichen oder eines anderen Partikels oder eine Bindung an ein Sauerstoffatom eines anderen Kerns darstellen. Auf diese Weise wird ein Netzwerk gebildet, beispielsweise wie in einem Silikat oder einem Alumosilikat. Alternativ können die freien Valenzen durch einen organischen Rest abgesättigt sein. Geeignete organische Reste sind Alkyl, Alkenyl, Cycloalkyl, Aryl, Arylalkyl, Alkylaryl, Alkoxy, eine Gruppe der Formel R¹COY-, worin R¹ den nach Entfernung der Carboxylgruppe verbleibenden Rest einer ethylenisch ungesättigten C₃-C₈-Monocarbonsäure oder C₄-C₈-Dicarbonsäure bedeutet und Y für O oder NR² steht, wobei R² für H oder C₁-C₄-Alkyl steht, oder eine phosphorhaltige, insbesondere phosphat-, pyrophosphat- und phosphitgruppen-haltige Gruppe.

Vorzugsweise sind die freien Valenzen von Me durch Alkyl, Aryl, Alkoxy, eine Gruppe der Formel R¹COY-, worin R¹ und Y die oben angegebenen Bedeutungen besitzen oder eine phosphorhaltige Gruppe abgesättigt, wobei Gruppen der Formel R¹COY- besonders bevorzugt sind.

Die Gruppe der Formel R¹COY- ist vorzugsweise abgeleitet von Acrylsäure, Methacrylsäure, Crotonsäure, Sorbinsäure, Vinylessigsäure, Maleinsäure, Fumarsäure, Itaconsäure oder Citraconsäure, wobei Acrylsäure und Methacrylsäure besonders bevorzugt sind.

Der Rest R (wenn vorhanden) stellt ein divalentes Bindeglied zur reaktionsfähigen funktionellen Gruppe X dar. R kann somit ein divalenter organischer Rest sein, der sich an ein Sauerstoffatom des Kerns A oder des Segmentes MeO oder an ein Metall oder Halbmetall Me einerseits und an die reaktionsfähige funktionelle Gruppe andererseits anbinden läßt. Im allgemeinen steht R für die oben bereits genannten Reste, wobei der Alkoxy-bzw. Acyloxyrest nicht mit dem Sauerstoffatom an eines der erwähnten Sauerstoffatome des Kerns oder der Gruppen MeO oder R(O) gebunden sein kann. Die Wahl der Gruppe R richtet sich nach den gewünschten Eigenschaften der Partikel und nach der Art der reaktionsfähigen funktionellen Gruppe X. Bevorzugte Reste R sind divalentes Alkyl, Hydroxyalkyl, Alkoxy, Acyloxy oder ein Rest, der nach Entfernung von zwei phenolischen Wasserstoffatomen von einer Phenolverbindung verbleibt, die mindestens zwei phenolische Hydroxylgruppen aufweist. Geeignete Phenolverbindungen sind Bisphenole, wie sie z. B. in Ullmann's Encyclopedia of Industrial Chemistry 1991, Vol. A 19, Seite 349 angegeben sind. Der Inhalt dieser Publikation ist durch Bezugnahme Teil der vorliegenden Anmeldung. Bevorzugt sind Bisphenol A, B oder F.

Weitere geeignete Phenolverbindungen sind polymere Phenolverbindungen, wie Resole, Novolake etc.

Wenn R für einen Phenolrest steht, sind y1 und z = 0.

Die reaktionsfähige funktionelle Gruppe soll in der Lage sein, chemische Umsetzungsreaktionen mit anderen funktionellen Gruppen einzugehen, die entweder in den Partikeln bereits vorhanden sind oder extern in Coreaktanten vorliegen. Insbesondere soll sie in der Lage sein, eine Polymerisation (inclusive Polykondensation und Polyaddition) einzugehen, so daß Vernetzung und/oder Härtung erfolgt. Reaktionsfähige Gruppen sind insbesondere Epoxygruppen, Isocyanatgruppen, Gruppen mit mindestens einem aktiven Wasserstoffatom oder Gruppen mit mindestens einer ethylenisch ungesättigten Doppelbindung. X kann direkt an B oder ein Sauerstoffatom des Kerns gebunden sein, beispielsweise kann eine Vinylgruppe an eine Alkylgruppe gebunden sein, so daß -(B)_{w}-X für eine Alkenylgruppe steht. X kann auch über ein Bindeglied Z an B gebunden sein. Z ist im allgemeinen O, NR², wobei R² für H oder C₁-C₄-Alkyl steht, OCO, COO, NHCO oder CONH. Bevorzugte Gruppen X mit einer ethylenisch ungesättigten Doppelbindung sind solche der Formel 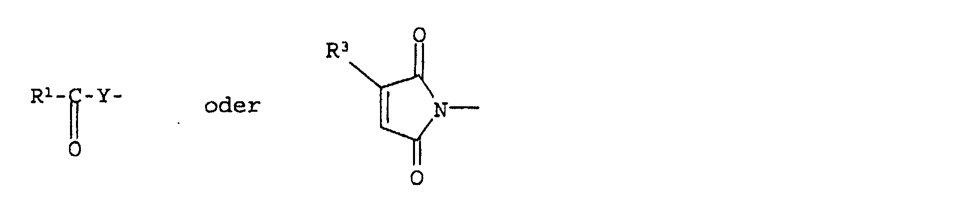 worin R¹ den nach Entfernung der Carboxylgruppe verbleibenden Rest einer ethylenisch ungesättigten C₃-C₈-Monocarbonsäure oder C₄-C₈-Dicarbonsäure bedeutet, Y für O oder NR² steht und R² und R³, die gleich oder verschieden sein können, für H oder C₁-C₄-Alkyl stehen. R¹ ist vorzugsweise abgeleitet von Acrylsäure, Methacrylsäure, Crotonsäure, Sorbinsäure, Vinylessigsäure, Maleinsäure, Fumarsäure, Itaconsäure und Citraconsäure. Ein Beispiel für X ist die Acryloder Methacrylgruppe, die vorzugsweise über O oder NH an B gebunden ist.

Eine bevorzugte Ausführungsform sind Partikel, bei denen der Kern A Siliciumdioxid, Titandioxid oder ein Si/Al-Mischoxid ist, - B-X für (MeO)ₓMe(O)_{y1} (CH₂)ₙOCOCR⁴ = CH₂ oder (MeO)ₓMe(O)_{y1}CH₂CHOHCH₂O0COCR⁴=CH₂ steht, wobei Me für Si, Al, Ti oder Zr, x für 1 oder 2, y1 für 0 oder 1, n für 2 bis 6 und R⁴ für H oder CH₃ steht, wobei die freien Valenzen von Si, Al, Ti oder Zr durch Alkoxyreste abgesättigt und/oder an Sauerstoffatome des gleichen oder eines anderen Kerns A gebunden sind.

Gruppen mit aktiven Wasserstoffatomen sind Hydroxylgruppen, primäre und sekundäre Aminogruppen, Thiolgruppen und Silanreste.

Eine Ausführungsform mit Silanresten sind Partikel, bei denen die Seitenketten durch einen Polyalkylhydrogensiloxanrest (Me = Si; x = 30-100; y1 und y2 = 0; X = H) gebildet werden. Derartige Partikel lassen sich beispielhaft anhand folgender Formel veranschaulichen: 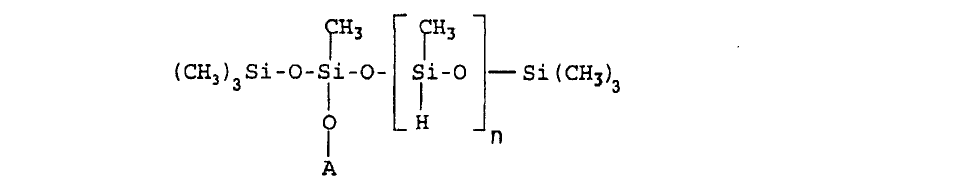
A = Kern
n = 30 bis 100, insbesondere 30 bis 50
A-O- kann dabei an ein beliebiges Silanatom gebunden sein. Aufgrund der mehreren Silaneinheiten können auch mehrere Kerne an die Siloxankette gebunden sein. Partikel dieser Art sind zur Umsetzung mit Siliconen und Epoxiden und als Haftungsverbesserer brauchbar.

Die erfindungsgemäßen Partikel besitzen in der Regel mehrere Seitenketten. Es ist dann möglich, Gruppen X mit unterschiedlicher Reaktivität einzubauen. Aufgrund der unterschiedlichen Reaktivität lassen sich die Eigenschaften der Partikel variieren, beispielsweise lassen sich Materialien mit dualen oder sogar multiplen Härtungsfunktionen herstellen.

Die erfindungsgemäßen Partikel sind im allgemeinen in Wasser unlöslich, sie können aber in Wasser oder anderen Medien, in welchen sie unlöslich sind, mittels üblicher Emulgatoren und/oder Schutzkolloide dispergiert werden. Sie können auch aus ihrer Schmelze verarbeitet werden, weil sie Schmelz- und Erweichungspunkte < 300 °C, vorzugsweise < 250°C, besitzen. Im Vergleich zu den entsprechenden mittels Sol-Gel-Prozeß hergestellten Produkten besitzen sie in der Schmelze eine wesentlich verbesserte "Heat History", weil der anorganische Kern die organischen Bestandteile im Makromolekül zumindest temporär schützt. Unter dem Terminus "Heat History" bei Schmelzmassen versteht der Fachmann alle die kritischen Parameter, die beim Aufschmelzen und in der Schmelze die organischen Bestandteile thermisch schädigen und damit die Endeigenschaften, wie z.B. Wärmestandfestigkeit, negativ beeinflussen. Sie hierzu R. Jordan "Schmelzklebstoffe" Bd. 4a (1985) und Bd. 4b (1986), Hinterwaldner-Verlag, München. - Mit den erfindungsgemäßen Partikeln läßt sich diese "Heat History" essentiell verbessern und die Thermosensibilität erheblich reduzieren, was vor allem beim Compoundieren und Applizieren der damit hergestellten Schmelzmassen von Vorteil ist.

Darüber hinaus sind die erfindungsgemäßen Partikel in inerten Lösemitteln, wie Aceton, Methylethylketon, Alkoholen (Methanol, Ethanol, Butanole, etc.), Essigsäureethylester etc., sowie in zahlreichen Coreaktanten löslich, die mit der reaktionsfähigen funktionellen Gruppe X zur Reaktion gebracht werden können. Beispielsweise sind die Partikel in einer großen Zahl ethylenisch ungesättigter Monomere, wie vinylaromatischen Verbindungen, beispielsweise Styrol, Estern der Acrylsäure oder Methacrylsäure mit C₁-C₁₂-Alkanolen oder C₁-C₁₂-Alkandiolen, z.B. Methyl(meth)acrylat, n-Butyl (meth)acrylat; t-Butyl(meth)acrylat, Ethylhexyl(meth)acrylat, Acrylnitril, Methacrylnitril, Acrylamid und Methacrylamid sowie die N-C₁-C₄-alkylierten Produkte davon, Vinyl-C₁-C₈-alkylether, Ester von Vinylalkohol mit C₁-C₁₂ - Alkancarbonsäuren, insbesondere Vinylacetat, Vinylpropionat, N-Vinyllactame, insbesondere N-Vinylpyrrolidon, C₂-C₆-Olefine, insbesondere Ethylen und Propylen, Butadien oder Isopren etc., zumindest soweit löslich oder dispergierbar, daß eine Copolymerisation mit den Monomeren vorgenommen werden kann. Durch die Copolymerisation entsteht ein polymeres Netzwerk, in das die Partikel kovalent eingebunden sind.

Die Eigenschaften der erfindungsgemäßen Partikel werden auch von der Art und dem Mengenanteil der Kernpartikel und der Metalle bzw. Halbmetalle Me in den Seitenketten bestimmt. Mit steigendem Gehalt an diesen. Komponenten steigt die Hochtemperaturbeständigkeit der Partikel bis Temperaturen oberhalb 350° C und sogar oberhalb 400° C. Es lassen sich Tg-Werte von bis zu 600° C erzielen. Die Temperaturbeständigkeit solcher Produkte liegt im allgemeinen 50 bis 100° C über dem jeweiligen Glasübergangspunkt. Besonders hochtemperaturbeständige Materialien erhält man, wenn man für die Metalle bzw. Halbmetalle in den Seitenketten Si, Ti und Zr kombiniert.

Die erfindungsgemäßen Partikel können durch geeignete Wahl der reaktionsfähigen Gruppen mit sich selbst zu Homopolymerisaten, vor allem aber mit anderen Reaktionspartnern zu Copolymerisaten umgesetzt werden, beispielsweise wenn man als Reaktionspartner die oben genannten ethylenisch ungesättigten Verbindungen einsetzt. Auf diese Weise lassen sich die Eigenschaften der erhaltenen Produkte nahezu beliebig variieren.

Die Homopolymerisation oder die Copolymerisation mit den Reaktionspartnern (Coreaktanten) erfolgt in üblicher, dem Fachmann bekannter Weise, beispielsweise durch radikalische Polymerisation, wenn X für ethylenisch ungesättigte Gruppen steht oder einer solche Gruppe aufweist und ethylenisch ungesättigte Monomere als Reaktionspartner verwendet werden. Geeignete Initiatoren für die Polymerisation sind beispielsweise organische Peroxide und Hydroperoxide, wie Benzoylperoxid, t-Butylhydroperoxid, Persalze, wie Natriumpersulfat, Natriumperoxodisulfat; Wasserstoffperoxid; Azoverbindungen,wie Azobisisobutyronitril etc. Die radikalische Copolymerisation kann auch durch Licht, beispielsweise UV-Strahlen oder Tageslicht, in Gegenwart von Fotoinitiatoren oder durch Elektronenstrahlen initiiert werden. Auch hitzehärtbare Systeme auf Basis von Epoxyden, ethylenisch ungesättigten Verbindungen und Isocyanaten kommen in Betracht.

Polyadditionssysteme liegen vor, wenn eine der Komponenten im System Epoxy- oder Isocyanatgruppen und die andere Komponente Gruppen mit aktiven Wasserstoffatomen aufweist. Beispielsweise können die erfindungsgemäßen Partikel, bei denen die reaktionsfähige Gruppe eine Epoxy- oder Isocyanatgruppe ist, mit Alkoholen oder primären oder sekundären Aminen, insbesondere Poyolen und Polyaminen, zur Reaktion gebracht werden.

Die Herstellung der erfindungsgemäßen Partikel erfolgt ausgehend vom Kern A, wobei das gewählte Oxid in fester, feinteiliger Form eingesetzt wird. Die mittlere Teilchengröße und die spezifische Oberfläche der Kernpartikel liegen im allgemeinen in den oben für die erfindungsgemäßen Partikel angegebenen Bereichen. Brauchbare Kernpartikel sind im Handel erhältich, beispielsweise als hochdisperse Kieselsäure, wie Aerosil® der Degussa AG, Frankfurt, HDK 80, 100 und 600 der Wacker-Chemie GmbH, München und Cab-O-Sil® der Cabot Corp., Boston, Mass., USA oder hochdisperses Titandioxid, wie Titandioxid P25 der Degussa AG. Auch die Mischoxide sind im Handel erhältlich, z.B. Si-Al-Mischoxide unter der Bezeichnung Aerosil® MOX und COK der Degussa AG.

Die Anbindung der Reste B, -B-X und/oder X erfolgt ausgehend von den Kernpartikeln in fester Form in einem Verfahrensschritt (in situ) in Gegenwart von starken Säuren, wie überraschenderweise gefunden wurde. Im allgemeinen wird das die Seitenkette -BX bzw. die Gruppe -B- bildende Material vorgelegt und die Kernpartikel werden eingearbeitet, beispielsweise durch Rühren. Zweckmäßigerweise wird dabei bei erhöhter Temperatur gearbeitet, die im allgemeinen im Bereich von 30 bis 80°C liegt. Alternativ können die Kernpartikel auch mit dem die Seitenketten bildenden Material imprägniert werden.

Falls erforderlich, wird zu dem erhaltenen Gemisch dann ein Reagens gegeben, das in der Lage ist, die Umsetzung mit den OH-Gruppen des Kerns A zu bewirken. Im allgemeinen handelt es sich dabei um eine starke Säure, einschließlich Lewis-Säuren, die als Katalysator die Umsetzung bewirkt. Die Menge an starker Säure liegt im allgemeinen im Bereich von 1 bis 10 Gew.-%, bezogen auf die Menge an Kernpartikel. Geeignete starke Säuren sind anorganische und organische Säuren, wie Schwefelsäure, Phosphorsäure, Maleinsäure, Methansulfonsäure oder p-Toluolsulfonsäure oder deren Anhydride. Geeignet sind auch dualfunktionelle Verbindungen, die mindestens eine Säuregruppe und mindestens eine funktionelle vernetzbare organische Gruppe in ihrem Molekül aufweisen. Beispiele hierfür sind die Ester der Phosphorsäure mit α,β-ethylenisch ungesättigten Carbonsäuren, wie Acrylsäure und Methacrylsäure. Zweckmäßigerweise gibt man ein Tensid (insbesondere ein anionisches oder nicht-ionisches Tensid) in einer Menge von im allgemeinen 0,1 bis 3 Gew.-%, bezogen auf die Menge an Kernpartikel, zu, um die Benetzung der Kernpartikel zu erleichtern.

Die Umsetzung wird bei der oben genannten Temperatur durchgeführt, die Reaktionszeit liegt im allgemeinen im Bereich von 10 Minuten bis 5 Stunden. Nach beendeter Reaktion wird die starke Säure mit einer Base, beispielsweise Natrium- oder Kaliumhydroxid, neutralisiert. Die dabei gebildeten Salze können gewünschtenfalls mittels Ionenaustauscher entfernt werden.

Die Anbindung der Seitenkette -B-X erfolgt vorzugsweise in einer Stufe durch Umsetzung mit einer Verbindung Y-B-X, wobei Y für eine Gruppe steht, die in der Lage ist, mit den Hydroxidgruppen auf der Oberfläche der Kernpartikel zu reagieren. Geeignete Gruppen Y sind beispielsweise Hydroxygruppen, Epoxygruppen, Halogene, metallorganische Gruppen, wie Trialkoxysilan- oder Trialkoxititan-Verbindungen, wobei die freie Valenz von Si und Ti durch die Gruppe B-X abgesättigt ist. Beipiele für brauchbare Verbindungen Y-B-X sind Acryl- oder Methacryloxypropyl-trimethoxysilan, Acryl- oder Methacryloxypropyl-trimethoxytitan, Glyzidylacrylat oder - methacrylat, Epoxide mit einer, insbesondere 2 oder mehreren Epoxygruppen, wie Glycidol, Mono- und Diepoxide auf Basis von Bisphenol, Novolak und Kresolen, 2,3-Epoxypropylurethan mit mindestens einer verkappten Isocyanatgruppe, 2,3-Epoxypropyl(meth)acrylat, Allylglycidylcarbonate, Glycidylcyanurate, wie Alkoxy-diglycidylcyanurate, Alkylglycidylether und Glycerylamine etc. MeO-Brücken in der Seitenkette können durch Zugabe der entsprechenden monomeren oder polymeren Metallalkoxylate oder der partiellen Hydrolyseprodukte davon, beispielsweise Tetramethoxysilan, Tetraethoxysilan, Tetrabutoxytitan, Dimethoxydisilanol, Polydimethoxysiloxan, Aluminiumisopropylat etc, eingeführt werden. Die für die Einführung der Seitenkette -B-X erforderlichen Ausgangsverbindungen sind im Händel erhältlich oder können in dem Fachmann bekannter Weise hergestellt werden. Auch die Ausgangsverbindung für die Herstellung der Partikel mit einer Polyalkylhydrogensiloxanseitenkette ist im Handel erhältlich als Baysilone-Öl MH 15 der Bayer AG.

Alternativ kann die Einführung der Seitenkette in zwei Stufen erfolgen, indem zunächst der Rest B eingeführt wird und im Anschluß daran die Gruppe X mit dem Rest B verbunden wird. Die Einführung des Restes B erfolgt durch Umsetzung der Kernpartikel mit einer Verbindung Y-B-Y', wobei Y die oben genannten Bedeutungen besitzt. Y' steht für eine Gruppe, die in der Lage ist, mit dem zur Einführung der Gruppe X verwendeten Reaktionspartner zu reagieren. Im allgemeinen hat Y' die gleichen bedeutungen wie Y. Geeignete Verbindungen Y-B-Y' sind beispielsweise Metallalkoxidverbindungen, wie Tetramethoxysilan, Tetraethoxysilan, Tetrabutoxytitan, Poly- (diethoxysiloxan), Poly(dimethoxysiloxan), Diethoxysiloxan-s-butylaluminat, Diethoxysiloxan-ethyltitanat, Poly- (dibutyltitanat), Poly(octylenglykoltitanat), sowie die in der DE 4020316 A beschriebenen Silicium-, Aluminium- und Titanverbindungen. Auch diese Verbindungen sind im Handel erhältlich (z.B. von Kenrich Petrochemicals Inc., Bayonne, N.J., USA oder Gelest, Inc., Tullytown, P.A., USA) oder in dem Fachmann bekannter Weise herstellbar.

Das erhaltene Produkt wird dann in einem weiteren Schritt mit einem Reagenz zur Einführung der funktionellen Gruppe X umgesetzt. Geeignete Reagentien sind beispielsweise Metallalkoxide, wobei eine Valenz des Metalls mit der funktionellen Gruppe abgesättigt ist. Beispiele für derartige Verbindungen sind Isopropyl-dimethacrylisostearoyltitanat, Alkoxy-tri(meth)acryltitanat, wobei Alkoxy für CH₃O-(C₂H₄O)₂ steht, sowie die entsprechenden Siliziumverbindungen etc.. Diese Verbindungen sind von der Firma Kenrich Petrochemicals, Inc., Bayonne, USA, erhältlich oder können in dem Fachmann bekannter Weise hergestellt werden.

Durch Zugabe von Metallalkoxiden, bei denen mindestens eine Valenz des Metalls durch einen von einem Alkoxid verschiedenen Rest abgesättigt ist, können weitere an Me gebundene organische Reste eingeführt werden. Hierfür brauchbare Verbindungen sind beispielsweise Isopropyl-triisoatearoyltitanat, Isopropyl-tri(dodecyl)benzolsulfonyltitanat, Isopropyl-tri(dioctyl)phosphatotitanat, Isopropyl(4-amino)benzalsulfonyl-di(4-dodecyl)benzolaulfonyltitanat, Isopropyl-tri(dioctyl)pyrophosphatotitanat, Isopropyl-tri(N-ethylendiamino)ethyltitanat, Di(dioctyl)pyrophosphat-oxoethylentitanat, Di(dioctyl)phosphato-ethylentitanat, Di(dioctyl)pyrophosphato-ethylentitanat, Di(butyl, methyl)pyrophosphato-ethylentitanat, Tetraisopropyl-di(dioctyl)phosphatotitanat, Tetraoctyl-di(ditridecyl)phosphitotitanat, Tetra (2,2-diallyloxymethyl) butyl-di (ditridecyl) phosphitotitanat, Dimethacryl-oxoethylentitanat, Neoalkoxy-trineodecanoyltitanat, Neoalkoxy-tri(dodecyl)benzolsulfonyltitanat, Neoalkoxy-tri(dioctyl)phosphatotitanat, Neoalkoxy-tri(dioctyl)pyrophosphatotitanat, Neoalkoxy-tri(N-ethylendiamino)ethyltitanat, Neoalkoxy-tri(m-amino)phenyltitanat, und die entsprechenden Zirkoniumverbindungen. Auch diese Verbindungen sind von Kenrich Petrochemicals, Inc. erhältlich.

Die erfindungsgemäßen Partikel sind hervorragende Rückgratpolymere und Rückgratbindemittel und bilden eine neue und innovative Stoffklasse. Sie lassen sich allein oder mit Coreaktanten zur Herstellung von Beschichtungs-, Überzugs-, Polymer-, Form-, Verguß-, Kleb- und Dichtmassen, Lacken, Oberflächenvergütungsmitteln, Massen für den Dental-, Kosmetik- und Medizinbereich und/oder als Bindemittel für Holzwerkstoffe und Steinmassen und dergleichen formulieren. Solche Formulierungen können mit üblichen Hilfsstoffen, wie
- Additiven, wie zähelastifizierende Stoffe-, Licht- und Alterungsschutzmittel, Weichmacher, Gleitmittel, Antistatika, Haftvermittler
- anorganischen und organischen Füllstoffen und Verstärkungsmitteln, wie Calciumcarbonat, Kaolin, Leicht- und Schwerspate, Siliciumoxide, Erdalkalioxide, Metalloxide und -pulver, Mikrohohlkörper, Ruße, Holzmehle, Fasern aus α-Cellulose, Glas, Polyamid, Polyester, Graphit und Kohlenstoff
- Pigmenten und Farbstoffen, wie Weißpigmente, Titandioxid, Farbruße, Azopigmente und dgl.
modifiziert werden.

Die erfindungsgemäßen Partikel lassen sich vorteilhaft in Beschichtungsmassen einarbeiten. Die erhaltenen Beschichtungen, Filme und Coatings besitzen je nach Vernetzungsdichte hervorragende mechanische und physikalische Eigenschaften. So wird die Kratzfestigkeit im Vergleich zu Materialien, die nach dem Sol-Gel-Prozeß erhalten wurden, signifikant verbessert. Auch die Gaspermeationswerte, z.B. gegenüber Sauerstoff und Stickstoff, sind bei Filmen aus den erfindungsgemäßen Partikeln deutlich verbessert.

Durch die Einlagerung der Partikel haben sich insbesondere auch Veränderungen viskoelastischer Kenngrößen im Vergleich zu den unmodifizierten Polymerproben ergeben. Bei einer harmonischen Wechselbeanspruchung einer (polymeren) Probe folgt die Dehnung - wie in vielen anderen physikalischen Ursache-Wirkungs-Beziehungen - der mechanischen Wechselspannung verzögert nach. Der im Hooke' schen Gesetz auftretende Elastizitätsmodul E, der ein Maß für den Widerstand von Materialien gegenüber mechanischen Beanspruchungen (Festigkeit) ist, ist deshalb komplex anzusetzen (E' + iE"), wobei der Speichermodul E' und der Verlustmodul E" temperatur- und frequenzabhängig sind. Auch für die Module E' und E" drücken sich die Dispersionsrelationen in Analogie etwa zu den komplexen Größen Permeabilität und Dielektrizitätskonstante in Kramers-Kronig-Beziehungen aus.

Die Module E' und E" sind mit der Dynamisch-Mechanischen Thermoanalyse (DMTA) zu bestimmen. Hierzu werden Folien, Schichten oder auch Fasern einer harmonischen Erregerschwingung im Bereich von 0,01 bis 200 Hz ausgesetzt und dabei gleichzeitig temperaturprogrammiert erwärmt.

Als abgeleitetete charakteristische Materialparameter erhält man den Dämpfungsfaktor tan δ = E''/E' sowie die Glasübergangstemperatur Tg, oberhalb der die Materialien erweichen. Die Tg ergibt sich aus der Lage des maximalen Verlustmoduls.

Die Ergebnisse der dynamisch-mechanischen Messungen sind den Anwendungsbeispielen zu entnehmen. Diese Ergebnisse können nur durch eine äußerst effiziente heterogene Copolymerisation zwischen den ausgedehnten Oberflächenbereichen der reaktiven Partikel und dem organischen Substrat erklärt werden (Erhöhung der polymeren Netzwerkdichte) . Damit geht eine überdurchschnittliche Verbesserung makroskopischer Eigenschaften wie z.B. Hochtemperaturstabilität, Kratzund Abriebfestigkeit sowie Verbundfestigkeit und Gasbarrierewirkung einher. Um diese Ergebnisse in ihrer Wirkung einschätzen zu können, ist auf die Untersuchungen von anderen polymeren Nanokompositen mit eingelagerten polymerisationsinaktiven Teilchen zu verweisen (T.Lan, T.J. Pinnavaia, Chem. Mater. 6 (1994) 2216, W. Helbert, J.Y. Cavafile, A. Dufresne, Polym. Composites 17 (1996) 604), wo eine signifikante Erhöhung des Speichermoduls nur im oberhalb von Tg liegenden Erweichungsbereich festgestellt werden konnte. Zudem bleibt in diesen Systemen die Glasübergangstemperatur durch den nanoglobularen Füllstoff weitgehend unbeeinflußt.

Durch die kovalente Einbindung der Partikel erhöht sich der Speichermodul im gesamten vermessenen Temperaturbereich beträchtlich.

Dadurch sind Temperaturbeständigkeiten und Verbundfestigkeiten bis zu 600° C und höher, extreme Kratz- und Abriebfestigkeiten, ausgeprägte Sperrwirkungen gegenüber Gasen, wie Stickstoff und Sauerstoff, sowie gute adhäsive Verbundfestigkeiten neben hoher Chemikalien-, Langzeit- und Alterungsbeständigkeit gegeben.

Die erfindungsgemäßen Partikel können allein und auch mit Coreaktanten geschäumt werden. Zum Herstellen solcher Schäume lassen sich bekannte chemische Treibmittel, wie Azoverbindungen, Azodicarbonamide, Hydrazin-Derivate, Semicarbazide, aber auch Gase, wie Stickstoff, Kohlendioxid, wasserstoffperoxid und andere anorganische und organische Perverbindungen, sowie Blähmittel, wie z.B. Calciumcarbid, die in Kontakt mit Wasser Gase bilden, einsetzen.

Die mit den erfindungsgemäßen Partikeln hergestellten Schäume besitzen herausragende Widerstandseigenschaften im Brandfalle, weil sie eine hohe Glasübergangstemperatur besitzen und durch den hohen Gehalt an anorganischen Bestandteilen nur begrenzt oder überhaupt nicht brennbar sind. Je nach Schaumart wirkt sich zusätzlich neben der hohen Temperaturbeständigkeit auch der sehr gute Isolationswert positiv aus.

Darüberhinaus entwickeln die Partikel an den Grenzflächen zwischen Fügeteiloberflächen und Klebstoffilm sehr hohe adhäsive Kräfte ohne die Kohäsionsfestigkeit der ausgehärteten Klebschicht - selbst bei hohen Temperaturen - zu beeinträchtigen.

Falls die Partikel phosphorhaltige Gruppen aufweisen, wird bessere Korrosions- und Wasserbeständigkeit erzielt.
Außerdem hat sich gezeigt, daß bei der Anwendung der erfindungsgemäßen Partikel in Zusammensetzungen überraschenderweise keine die Rheologie verbessernden Additive und bei mit Mineralstoffen gefüllten Systemen keine Thixotropier- und Antisedimentationsmittel erforderlich sind. Die Transparenz der Zusammensetzungen wird selbst bei hohen Gehalten an Partikeln nicht negativ beeinflußt.

### Beispiele

Die Beispiele erläutern die Erfindung ohne sie zu beschränken. Die in den Beispielen verwendeten Abkürzungen haben folgende Bedeutungen:

| **Partikel Kern A** | | | |
|---|---|---|---|
| **Rohstoff** | **Abkürzung** | **Partikelgröße** (mittlere) | **Oberfläche nach BET** (DIN 6613 1) m²/g |
| Hochdisperse Kieselsäure | HDK 600 | 40 nm | 200 ± 50 |
| | HDK 100 | 0,5mm | - |
| Mischoxid (98,3 % SiO2 + 0,3-1,3 % Al₂O₃) | HDK 80 | 30 nm | 80 ± 20 |
| Aluminiumoxid | HDA | 13 nm | 100 ± 15 |
| Titandioxid | HDT | 21 nm | 50 ± 15 |

| **Partikel Seitenketten B-X** | |
|---|---|
| **Rohstoff** | **Abkürzung** |
| Methacryloxypropyltrimethoxysilan | MEMO |
| 3-Aminopropyltrimethoxysilan | APMO |
| Vinyltrimethoxysilan | VTMOS |
| Vinyltriethoxysilan | VTEOS |
| Oligomeres alumo-siloxan-modifiziertes Methacryloxypropyltrimethoxysilan | MEMO AL |
| Polymethyl-H-Siloxan mit ca. 40 -Si-H-Gruppen | MH 15 |

| Aluminiumisopropylat, | |
|---|---|
| 97 Gew.-% | ALUPROP |
| Glycidylmethacrylat | GMA |
| Zirkonium-IV-ethylat | ZIRKO |

| **Comonomer / Reaktiver Löser für Partikel** | |
|---|---|
| **Rohstoff** | **Abkürzung** |
| Tetraethoxypentaerythrittetraacrylat | Mo 10 |
| Bisethoxy-bis-phenol A-diacrylat gelöst in Tripropylenglykoldiacrylat | Mo 20 |
| Trisethoxy-trimethylolpropantriacrylat | TETMPTA |
| 1,2-Epoxycyclohexan-4-carbonsäure-4'-(1',2'-epoxycyclohexyl)-methylester | Mo 30 |
| Trimethylolpropantriacrylat | TMPTA |
| Trisethoxy-2,4,6-triamino-s-triazintriacrylat | Viaktin 5970 |

| **Diverses** | |
|---|---|
| Netzmittel | wäßrige Natriumdodecylsulfatlösung, 30 Gew.-% -ig |
| Katalysator | wäßrige Methansulfonsäure, 70 Gew.-% -ig (Beispiele 1-11) Maleinsäure bzw. Maleinsäureanhydrid (Beispiele 12-16) |

- GT: Gewichtsteile
- ESH: Elektronenstrahlhärtung

### Herstellung

Die Rezepturen der Beispiele 1 bis 15 sind in Tabelle 1 zusammengefaßt.

### Beispiele 1 bis 15:

Die Rohstoffe und Zusammensetzungen der erfindungsgemäßen Partikel gehen aus Tabelle 1 hervor, während die Herstellung nachstehend beschrieben ist.

In einem Rührgefäß wird das jeweilige Comonomere vorgelegt und auf die vorgegebene Reaktionstemperatur erwärmt. Bei Erreichen der Reaktionstemperatur werden die angegebenen Mengen an Kernmaterial A und die Verbindungen B-X unter intensivem Rühren alternierend in das flüssige Comonomer eingetragen und homogen verteilt. Anschließend erfolgt unter Rühren während 15 Minuten der Zusatz von Wasser und der Netzmittel- und Katalysatormengen gemäß Tabelle 1. Anschließend wird bei der jeweiligen Reaktionstemperatur weitergerührt. Zum Schluß neutralisiert man gegebenenfalls mit 50 %-iger wäßriger Natriumhydroxidlösung während etwa 15 Minuten und kühlt das Reaktionsgemisch auf Raumtemperatur ab.
Im Beispiel 11 wird kein Comonomer als Vorlage benötigt.
Hier wird MH 15 vorgelegt.

### Beispiel 16:

In einem Rührgefäß (2 1), ausgerüstet mit Rührwerk, Tropftrichter und Rückflusskühler werden 100 GT HDK 600 und/oder HDA in 1 1 Aceton vorgelegt. Das Gemisch wird unter intensivem Rühren zum Sieden erhitzt. Anschließend werden unter fortwährendem Rühren 50 GT MEMO und danach 1,5 GT Maleinsäureanhydrid, gelöst in 10 GT Wasser, zugesetzt. Man erhitzt noch 2 Stunden unter Rückfluss weiter. Zum Schluss wird das Lösungsmittel bei 30 °C unter vermindertem Druck (1,6 kPa) abdestilliert. Das erhaltene Nanopulver wird anschließend zerkleinert bzw. mikronisiert. Die Ausbeute ist nahezu quantitiv.

### Beispiel 17:

30,0 GT HDK 100 wurden in 10,0 GT GMA angeteigt und anschließend mit 60,0 GT eines Gemisches bestehend aus 12 Gew.-% TMPTA und 88 % TETMPTA vermischt. Dieses Gemisch wird 45 Minuten in einer Fritsch Planetenkugelmühle "Pulverisette 5" unter Verwendung von Zirkonium-(IV)-oxid-Kugeln (4 Kugeln Ø 20mm; 15 Kugeln Ø 10 mm; Mahlbechervolumen 75 ml) bei einer Rotationsgeschwindigkeit der Mahlbecher von 360 min-1 gemahlen. Anschließend tropft man 0,2 ml 70 Gew.-%-ige wäßrige Perchlorsäure zu, damit die kovalente Anbindung über die Epoxygruppe des GMA an das Kernmaterial A erfolgt. Nach weiterer etwa 20-minütiger Mahlung ist die erhaltene kolloidale Dispersion nach bekannten Methoden verarbeitbar. Hinsichtlich der Eigenschaften siehe Tabelle 2.

### Beispiel 18:

In einem Rührgefäß mit aufgesetztem Rückflußkühler werden 46 GT HDK 600 und 34 GT MEMO in 200 GT Aceton gelöst und zum Sieden erwärmt. Anschließend werden unter intensivem Rühren schnellstens 20 GT ALUPROP zudosiert. Nach etwa 5 Minuten erfolgt die Zugabe von 13 GT Wasser, 2,45 GT Netzmittel- und 1,3 GT Katalysatorlösung innerhalb von 15 Minuten. Bei 56 °C wird eine Stunde weitergerührt. Anschließend wird das Reaktionsprodukt gegebenenfalls mit 50 %iger Natriumhydroxidlösung neutralisiert und das inerte Lösemittel unter Vakuum abdestilliert. Die reine Reaktionsmasse ist bei Raumtemperatur halbfest bis fest und besitzt thermoplastische Eigenschaften.

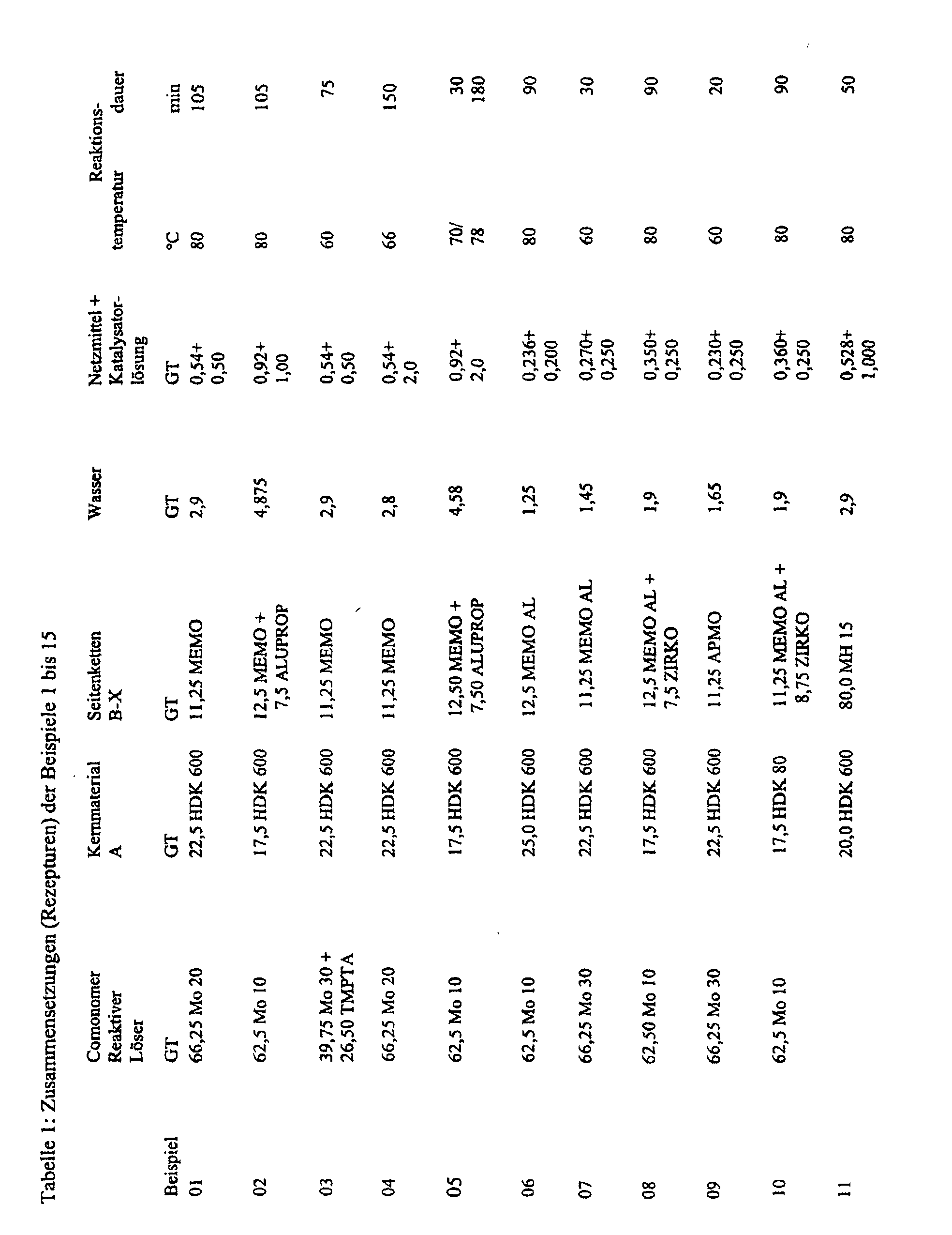

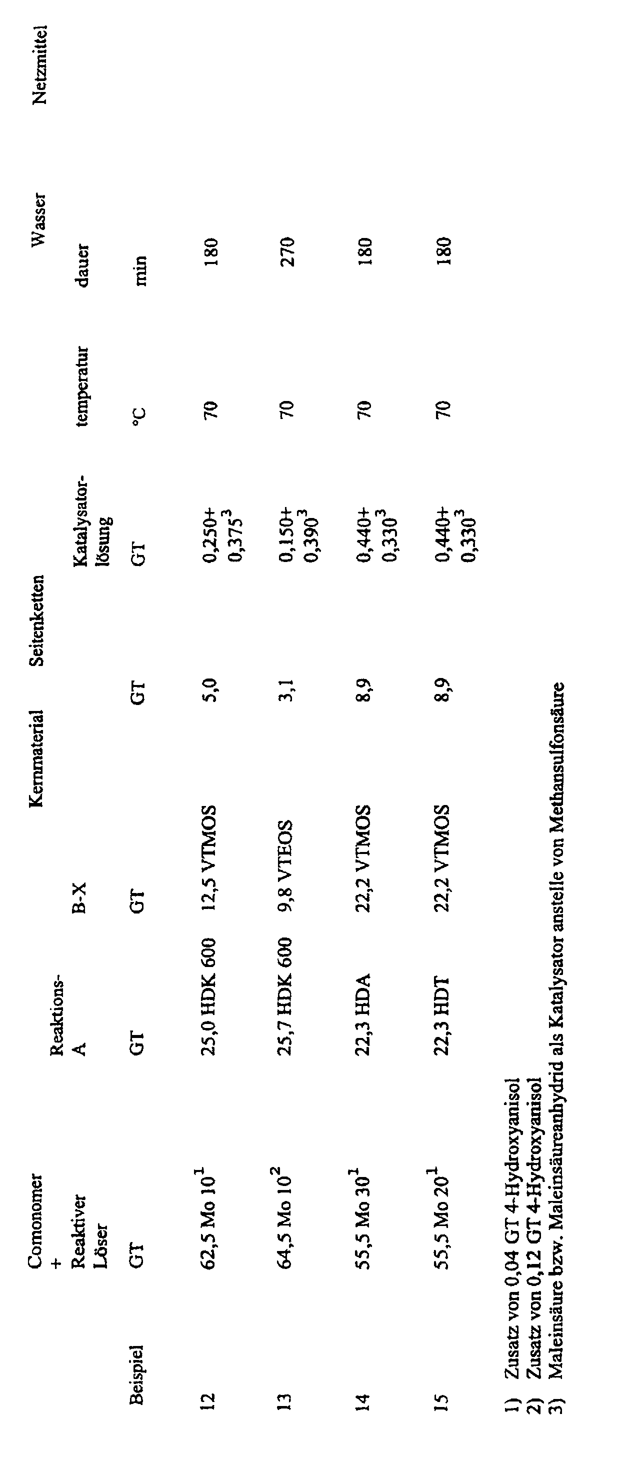

### Vergleichsbeispiel 1

### "Sol-Gel-Prozeß":

In einem Dreihalskolben mit Rührer und Gasdurchleitung werden 40 ml TMPTA vorgelegt. Nach einstündiger kräftiger Spülung mit Reinstickstoff wird eine Lösung bestehend aus 0,5 GT Natrium in 10 ml absolutem Ethanol innerhalb von 5 Minuten zugesetzt. Nach weiterem 10-minütigem Rühren dosiert man 7,5 ml wasserfreies 2-Aminoethanol innerhalb von 20 Minuten zu. Anschließend wird noch 4 Stunden bei 50 °C unter ständiger N₂- Ein- und Durchleitung weitergerührt. Danach gibt man innerhalb von 15 Minuten 10 ml Orthokieselsäureethylester zu. Sodann wird zum Reaktionsgemisch eine Lösung von 0,25 g Netzmittel (Natriumdodecylsulfat gelöst in 5 ml Wasser) innerhalb einer Stunde zugetropft. Es wird noch eine Stunde bei 50 °C weitergerührt. Zum Schluß wird der Ansatz so schnell wie möglich auf Raumtemperatur abgekühlt. Dieses Reaktionsprodukt wurde im Vergleich mit den erfindungsgemäßen Partikeln geprüft (Tabelle 3).

### vergleichsbeispiel 2

### "Sol-Gel-Lack"

In einem Rührgefäß werden 33 GT TMPTA und 0,08 GT 4-Hydroxyanisol vorgelegt und auf 65-70 °C erwärmt. Zum vorgewärmeten Acrylatgemisch wird eine Lösung aus 0,2 GT Maleinsäureanhydrid, 0,7 GT Natriumdodecylsulfat und 14,0 GT Wasser zugegeben und anschließend innerhalb 30 Minuten ein Gemisch aus 45,5 GT Tetraethoxysilan und 6,5 GT MEMO zugegeben. Anschließend rührt man das Reaktionsgemisch weiter und destilliert innerhalb von 6 Stunden das Wasser/Alkohol-Gemisch bei einem Druck von 1,6 kPa ab. Zuletzt kühlt man die verbleibende Reaktionsmasse schnellstens auf Raumtemperatur ab.

### Anwendungsbeispiele 19-21

### Beispiel 19:

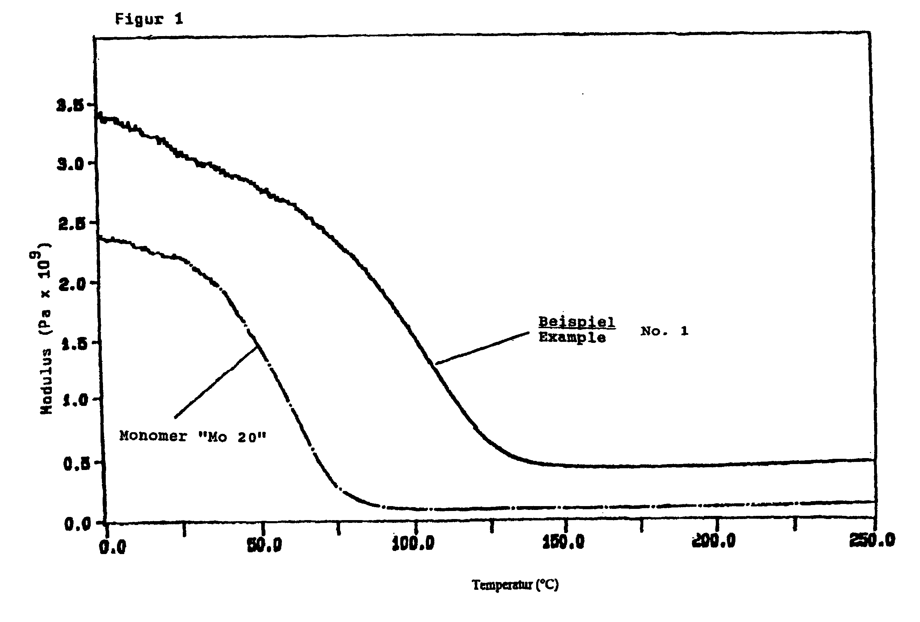

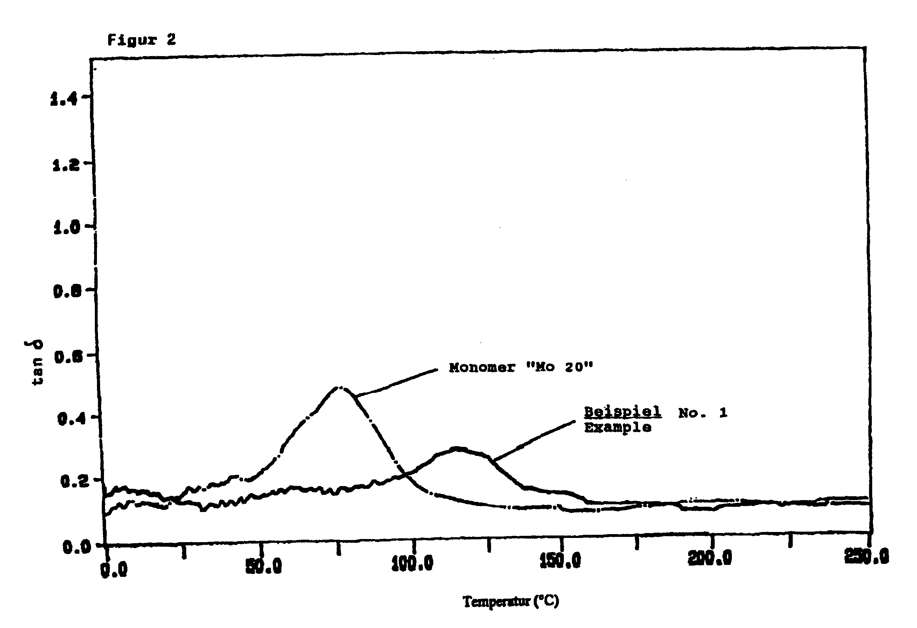

Mit der kolloidalen Dispersion aus Beispiel 1 ist mit einem Rakel ein etwa 0, 1 mm starker Film auf einem Silikon-Trennpapier hergestellt worden. Dieser Film ist anschließend mit Elektronenstrahlen (Dosis 80 kGy/180 keV-Anlage) unter Schutzgasatmosphäre (N2) gehärtet worden. Dieser Film wurde der Dynamisch-Mechanischen Thermoanalyse (DMTA) unterzogen, um den Dämpfungsfaktor tan δ = E" / E' und die Glasübergangstemperatur (Tg) zu bestimmen. Die Messungen wurden mit der Perkin Eimer-Anlage DMA 7e mit angelegten statistischen und dynamischen Erregerkräften von 200 mN bei einer Frequenz von 1 Hz im Temperaturbereich von -20 bis +250 °C durchgeführt. In Fig. 1 ist zu erkennen, daß der Speichermodul für die erfindungsgemäßen Partikel im Vergleich zum reinen Acrylat "Mo 20" bereits im unterhalb von Tg liegenden Erstarrungsbereich deutlich erhöht ist (bei 20 °C um etwa einen Faktor 1,4). Im mechanischen Dispersionsgebiet tritt eine Verschiebung des Maximums von tan δ ein und die Glasübergangstemperatur (T_{g}) liegt um einige 10 K höher (Fig. 2).

### Beispiel 20:

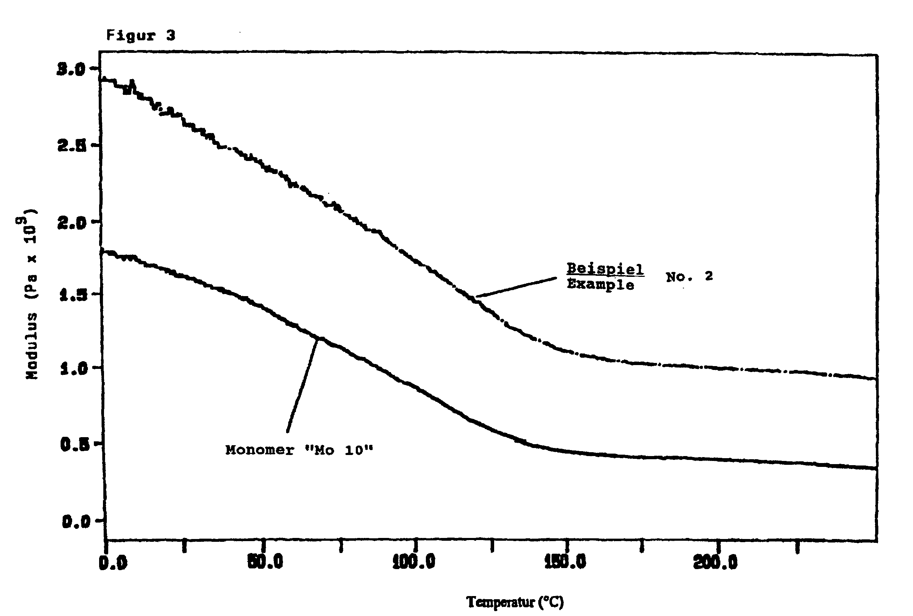

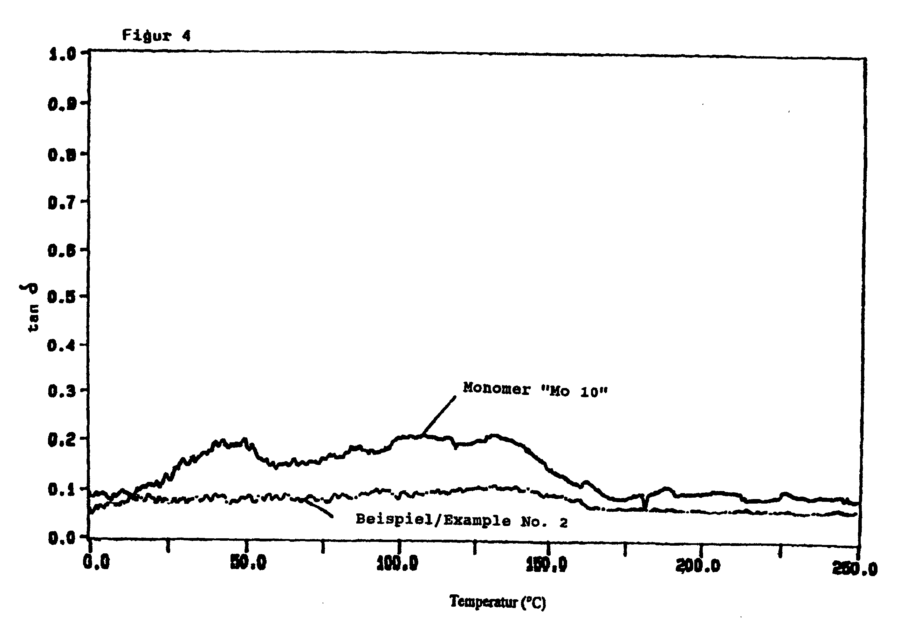

Mit der kolloidalen Dispersion aus Beispiel 2 wurde analog Beispiel 19 verfahren und ein Testfilm hergestellt. Diese Dispersionen unterscheiden sich nur durch ihre Comonomere. Allein der Austausch des Comonomeren "Mo 20" (Beispiel 1) durch das Comonomere "Mo 10" demonstriert erfindungsgemäß eine weitere Verbesserung der viskoelastischen und makroskopischen Eigenschaften, die durch die Modifizierung des hochvernetzenden Tetraacrylats "Mo 10" erreicht wird und keinen Glasübergang mehr besitzt. Fig. 3 zeigt bereits beim unmodifizierten organischen Substrat eine weitaus höhere Temperaturstabilität des Speichermoduls im Vergleich zu "Mo 20" im Beispiel 1 (Fig. 1).
Durch die kovalente Einbindung der erfindungsgemäßen Partikel (Beispiel 2) erhöht sich der Speichermodul im gesamten vermessenen Temperaturbereich beträchtlich. Deshalb sind Temperaturbeständigkeiten von 600 °C und höher, extreme Kratz- und Abriebfestigkeiten, ausgeprägte Sperreigenschaften gegenüber Gasen, wie Stickstoff und Sauerstoff, sowie gute adhäsive Verbundfestigkeiten neben hoher Chemikalien-, Langzeit- und Alterungsbeständigkeiten gegeben und realisierbar (Fig. 4). Weitere Eigenschaften im Vergleich zum Monomer "Mo 10" und Vergleichsbeispiel 1 (Sol-Gel-Prozeß) sind in Tabelle 3 zusammengefaßt.

### Beispiel 21:

Mit der kolloidalen Dispersion aus Beispiel 2 wurde eine Klebmasse mit folgender Zusammensetzung formuliert:
50,0 GT Reaktive Dispersion nach Beispiel 2
44,0 GT Füllstoff "Calciumcarbonat", gecoatet
5,0 GT Dibenzoylperoxidpaste, 50 %-ig in Dioctylphthalat
1,0 GT N,N'-Diethylanilin

Zunächst wird in der reaktiven Dispersion das N, N - Diethylanilin (Beschleuniger) homogen verteilt. Anschließend wird der Füllstoff bei leicht erhöhter Temperatur (40 °C) homogen eingearbeitet. Zum Schluß setzt man den Reaktionsinitiator "Benzoylperoxidpaste" zu und verteilt ihn gleichmäßig in der Paste. Die fertige Masse hat eine Topfzeit (DIN 16920) von 25 Minuten/20 °C. Mit dieser Masse wurden entfettete Stahlprüfkörper in einschnittiger Überlappung für den Zugscherversuch nach DIN 53283 geklebt. Nach 24-stündiger Lagerung von 10 Prüfkörpern bei Raumtemperatur wurden sie geteilt; d.h. 5 dieser Prüfkörper wurden 48 Stunden bei 200 °C im Trockenschrank und anschließend 24 Stunden bei Raumtemperatur gelagert. Die anschließende Prüfung nach DIN 53283 ergab folgende Durchschnittswerte:

| Zugscherfestigkeit | |
|---|---|
| Härtung 24 Std. / 20 °C | 18 N / mm² |
| Härtung 24 Std. / 20 °C und | 17 N / mm² |
| Alterung 48 Std. / 200 °C | |

Die hohe Glasübergangstemperatur der gehärteten Partikeldispersion (Fig. 4) bestätigt die hohe Temperaturbeständigkeit solcher Massen ohne dabei Verlust an Verbundfestigkeiten in Kauf nehmen zu müssen. Die Klebschicht zeigte einen 100 %-igen Kohäsionsbruch.

**Tabelle 2:**

| Härte, Kratz- und Abriebfestigkeit der Beschichtung nach Beispiel 12 im Vergleich zu 2 Handelsprodukten | | | | | |
|---|---|---|---|---|---|
| | ESH-Dosis | Pendelhärte | Erichsen-Tiefung¹ | Diamant-nadelnach Vieardt² | Taber-Abraser Sandfall Methode |
| Produkt | [kGy] | [S] | [N] | [N] | [∼ 10 % - total) |
| Esa Lux LR 1283 (Handelsprodukt) - Vergleich - | 90 | 142 | 14/16 NH³ | 2 | 1400 - 3300 U |
| Viaktin 5970 (Handelsprodukt) - Vergleich - | 140 | 121 | ― | ― | 1500 - 2500 U |
| Beispiel 19 erfindungsgemäß | 60 | 144/152 NH³ | > 20 | 8 | 4500 - 9500 U |

| | | | | | |
|---|---|---|---|---|---|
| ¹Erichsen = Stahlblech | | | | | |
| ² Vicardt = Diamant | | | | | |
| ³ NH = Nachhärtung nach 12 Tagen | | | | | |

**Tabelle 3:**

| Eigenschaftsvergleiche mit Produkten nach dem Stand der Technik, dem Sol-Gel-Prozeß und dem Beispiel 2 (erfindungsgemäß) | | | | | | |
|---|---|---|---|---|---|---|
| | ESH-Dosis | Mikroritzhärte (Diamant) | Erichsen Härte DIN 55350 (Prüfstab 318) | Traber Abraser-Test DIN EN 438-2 | Glanz Einstrahlwinkel DIN/ISO 900ff | Gaspermation² ml/h XM² x bar DIN 53380 |
| Produkt | [kGy] | [N] | [N] | [U] | 60°C¹N₂ | Trenn-0₂ faktor |
| Monomer Mo 10 | 40 | 3,5 | 4 | 2700 | 75,0/39,8 8.4 | 22.3 2,7 |
| Vergleichsbeispiel 1 "Sol-Gel Prozeß" | 40 | 5,0 | 5,5 | 5200 | 68,0/54,9 4,3 | 17,2 4,0 |
| Beispiel 2 erfndungsgemäß | 40 | 8,0 | 7,5 | 8300 | 81,6/80,4 1,1 | 7,8 7,1 |

| | | | | | | |
|---|---|---|---|---|---|---|
| ¹Ausgangswert und nach 60 manuellen Kratzzyklen mit Stahlwolle | | | | | | |
| ² Papier (60 g) Auftragsgewicht 15 g / m² | | | | | | |

## Patentansprüche

1. Hochtemperaturbeständige polymerisierbare Metalloxidpartikel mit einer Glasübergangstemperatur der Homopolymerisate von ≥ 100 °C und mit einem Kern A aus mindestens einem Oxid eines Metalls oder Halbmetalls der dritten bis sechsten Hauptgruppe, der ersten bis achten Nebengruppe des Periodensystems oder der Lanthaniden und mit mindestens einer über ein oder mehrere Sauerstoffatome des Oxids kovalent an den Kem gebundenen Gruppe -(B)w-X, worin w für 0 oder 1 steht und B für einen Rest der Formeln
- (MeO)x Me(O)y1 - (R)y2-
oder
-R(O)z -
steht, wobei x für 0 bis 100, y1, y2 und z unabhängig voneinander für 0 oder 1 stehen und Me ein Metall oder Halbmetall der dritten bis sechsten Hauptgruppe oder der dritten bis achten Nebengruppe des Periodensystems bedeutet, wobei die freien Valenzen von Me eine Bindung an ein weiteres Sauerstoffatom des Kerns A und/oder eine Bindung Ober ein Sauerstoffatom an ein Me in einer anderen Gruppe B und/oder eine Bindung an ein Sauerstoffatom eines anderen Kerns A darstellen und/oder durch H, einen organischen Rest und/oder einen Triaikylsilyloxyrest abgesättigt sind,
R für divalentes Alkyl, Cycloalkyl, Aryl, Arylalkyl, Alkylaryl, Alkoxy, Acyl, Acyloxy oder für einen Rest steht, der nach Entfernung von zwei phenolischen Wasserstoffatomen aus einer mindestens zwei phenolische Hydroxylgruppen aufweisenden Phenolverbindung verbleibt, wobei R gegebenenfalls durch 1, 2 oder 3 Reste substituiert, die unabhängig voneinander ausgewählt sind unter Hydroxy, Alkoxy, Halogen und, im Falle von Aryl- oder Cycloalkylresten, auch Alkyl, und/oder durch ein oder zwei Sauerstoffatome in der Kette unterbrochen sein kann, und
X über ein Bindeglied Z, das ausgewählt ist unter O, NR², OCO, COO, NHCO oder CONH, wobei R² für H oder C₁ - C₄-Alkyl steht, an B gebunden sein kann und eine reaktionsfähige funktionelle Gruppe oder einen Rest mit einer reaktionsfähigen, funktionellen Gruppe bedeutet,
wobei die Gruppe oder die Gruppen -(B)_{w}-X mindestens 10 Gew.-%, bezogen auf das Gesamtgewicht der Partikel, ausmachen,
erhältlich durch kovalente Anbindung der Reste B, B-X und/oder X an den in fester Form vorliegenden Kern A in Gegenwart einer starken Säure.

2. Partikel nach Anspruch 1, wobei die Gruppe oder die Gruppen -BX mindestens 20 Gew.-%, bezo- gen auf das Gesamtgewicht der Partikel, ausmachen.

3. Partikel nach Anspruch 1 oder 2, wobei der Kern ein Si-, Al-, Ti- oder Zr-Oxid, oder ein Gemisch oder Mischoxid davon ist.

4. Partikel nach einem der vorhergehenden Ansprüche, wobei Me für ein Metall oder Halbmetall der dritten oder vierten Hauptgruppe oder der vierten Nebengruppe des Periodensystems steht.

5. Partikel nach Anspruch 4, wobei Me für Si, Ti, Al oder Zr steht.

6. Partikel nach einem der vorhergehenden Ansprüche, wobei B ausgewählt ist unter
a) - Me (O)_{y1}-R-
b) - Me-O-Me(O)_{y1}-R-
c) -Me-O-Me-O-Me(O)_{y1}-R-
d) -R-O-
e) -R -
worin y1 für 0 oder 1 steht und R die in Anspruch 1 angegebenen Bedeutungen besitzt.

7. Partikel nach Anspruch 6, wobei -Me-O-Me- im Rest b ausgewählt ist unter
- Si-O-Si-
- Si-O-Al-
- Si-O-Ti-
- Si-O-Zr-
- Al-O-Ti-
- Al-O-Zr-
- Al-O-Al-
und -Me-O-Me-O-Me- im Rest c für -Si-O-Ti-O-Zr- steht.

8. Partikel nach einem der vorhergehenden Ansprüche, wobei R für divalentes Alkyl, Hydroxyalkyl, Alkoxy oder Acyloxy steht oder ein nach Entfernung der beiden phenolischen Wasserstoffatome verbleibender Bisphenol A, B oder F-Rest.

9. Partikel nach einem der vorhergehenden Ansprüche, wobei X für eine Epoxygruppe, Isocyanatgruppe, eine Gruppe mit mindestens einem aktiven Wasserstoffatom oder eine Gruppe mit mindestens einer ethylenisch ungesättigten Doppelbindung steht.

10. Partikel nach Anspruch 9, wobei X für eine Epoxygruppe, eine Aminogruppe oder eine Gruppe der Formel 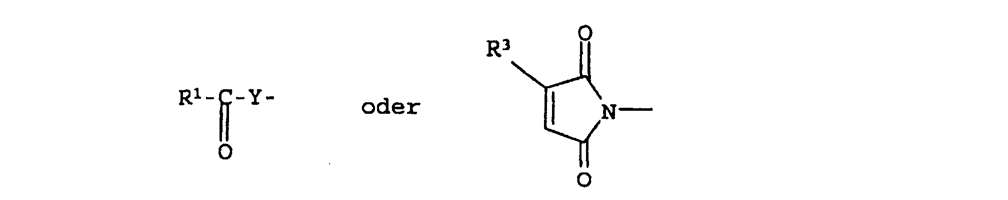 steht, worin R¹ den nach Entfernung der Carboxylgruppe verbleibenden Rest einer ethylenisch ungesättigten C₃-C₈-Monocarbonsäure oder C₄-C₈-Dicarbonsäure bedeutet und Y für O oder NR², wobei R² und R³ für H oder C₁-C₄-Alkyl stehen.

11. Partikel nach Anspruch 9 oder 10, wobei R für -(CH₂)ₙ-, - CH₂CHOHCH₂- oder -CH₂CHOKCH₂-Z- steht, worin n für 1 bis 6 und Z für einen nach Entfernung der beiden phenolischen Wasserstoffatome verbleibenden Bisphenolrest steht.

12. Partikel nach Anspruch 1, wobei
A Siliciumdioxid, Titandioxid oder ein Si/Al-Mischoxid ist,
- B-X für (MeO)ₓMe(O)_{y1}(CH₂)ₙOCOCR₄ = CH₂ oder (MeO)ₓMe(O)_{y1}CH₂CHOHCH₂OCOCR⁴ = CH₂ steht, wobei Me für Si, Al, Ti oder Zr, x für 1 oder 2, y1 für 0
oder 1, n für 2 bis 6 und R⁴ für H oder CH₃ steht,
wobei die freien Valenzen von Si, Al, Ti oder Zr
durch Alkoxyreste abgesättigt und/oder an Sauerstoffatome des gleichen oder eines anderen Kerns A gebunden sind.

13. Partikel nach Anspruch 12, wobei MeOMe bzw. MeOMeOMe die in Anspruch 7 angegebenen Bedeutungen besitzt.

14. Partikel nach einem der vorhergehenden Ansprüche, wobei die freien Valenzen von Me durch Alkyl, Aryl, Alkoxy, eine Gruppe der Formel 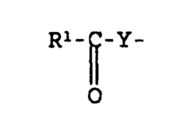 worin R¹ und Y die in Anspruch 10 angegebenen Bedeutungen besitzen, oder eine phosphathaltige Gruppe abgesättigt sind.

15. Partikel nach Anspruch 1, wobei die Seitenkette ein Polyalkylhydrogensiloxanrest ist.

16. Partikel nach einem der vorhergehenden Ansprüche mit einer Glasübergangstemperatur der Homopolymerisate von > 250° C.

17. Verfahren zur Herstellung der Partikel nach einem der Ansprüche 1 bis 16, **dadurch gekennzeichnet, dass** man die Reste B, -B-X und/oder X an den in fester Form vorliegenden Kern A in Gegenwart von starken Säuren kovalent anbindet.

18. Verfahren nach Anspruch 17, **dadurch gekennzeichnet, dass** man die kovalente Anbindung in einem Verfahrensschritt (in situ) durchführt.

19. Zusammensetzung, enthaltend die Partikel nach einem der Ansprüche 1 bis 16.

20. Verwendung der Partikel nach einem der Ansprüche 1 bis 16 zur Herstellung von als Beschichtungsmassen, Überzugsmassen, Klebmassen, Dichtmassen, Formmassen, Gußmassen, Polymermassen oder Massen für den Dental-, Kosmetik- oder Medizinbereich, Lacken, Oberflächenvergütungsmitteln oder als Bindemittel für Holzwerkstoffe und Steinmassen.

## Claims

1. High temperature resistant polymerizable metal oxide particles having a glass transition temperature of the homopolymers of ≥ 100°C and having a core A comprising at least one oxide of a metal or semimetal from main groups three to six, transition groups one to eight of the periodic system, or the lanthanides and having at least one group - (B)_{w}-X, which is bonded covalently to the core by way of one or more oxygen atoms of the oxide, w being 0 or 1 and B being a radical of the formulae
- (MeO)ₓ Me(O)_{y1} -(R)_{y2}- or -R(O)_{z}-
in which x is from 0 to 100, y1, y2 and z independently of one another are 0 or 1, and Me is a metal or semimetal from main groups three to six or transition groups three to eight of the periodic system, the free valances of Me representing a bond to a further oxygen atom of the core A and/or a bond via an oxygen atom to an Me in another group B and/or a bond to an oxygen atom of another core A and/or being satisfied by H, an organic radical and/or a trialkylsilyloxy radical;
R is divalent alkyl, cycloalkyl, aryl, arylalkyl, alkylaryl, alkoxy, acyl, acyloxy or a radical remaining following the removal of two phenolic hydrogen atoms from a phenol compound having at least two phenolic hydroxyl groups, it being possible for R to be substituted, if desired, by 1, 2 or 3 radicals selected independently of one another from hydroxy, alkoxy, halogen and also, in the case of aryl or cycloalkyl radicals, alkyl, and/or interrupted in the chain by one or two oxygen atoms, and
X may be attached to B via a link Z which is selected from O, NR², OCO, COO, NHCO or CONH, where R² is H or C₁-C₄ alkyl and is a reactive functional group or a radical containing a reactive functional group, wherein the group or groups - (B)_{w}-X account for at least 10 % by weight, based on the overall weight of the particles,
obtainable by covalent attachment of radicals B, B-X or X to the core A, present in solid form, in the presence of a strong acid.

2. The particles as claimed in claim 1, where the group or the groups -BX account for at least 20% by weight, based on the overall weight of the particles.

3. The particles as claimed in claim 1 or 2, where the core is an Si, Al, Ti or Zr oxide, or a mixture or mixed oxide thereof.

4. The particles as claimed in one of the preceding claims, where Me is a metal or semimetal from main group three or four or transition group four of the periodic system.

5. The particles as claimed in claim 4, where Me is Si, Ti, Al or Zr.

6. The particles as claimed in one of the preceding claims, where B is selected from
a) - Me (O)_{y1}-R-
b) - Me-O-Me (O)_{y1}-R-
c) -Me-O-Me-O-Me(O)_{y1}-R-
d) -R-O-
e) -R-
in which y¹ is 0 or 1 and R has the definitions indicated in claim 1.

7. The particles as claimed in claim 6, where -Me-O-Me- in the radical b is selected from
- Si-O-Si-
- Si-O-Al-
- Si-O-Ti-
- Si-O-Zr-
- Al-O-Ti-
- Al-O-Zr-
- Al-O-Al-
and -Me-O-Me-O-Me- in the radical c is -Si-O-Ti-O-Zr-.

8. The particles as claimed in one of the preceding claims, where R is divalent alkyl, hydroxyalkyl, alkoxy or acyloxy or a bisphenol A, B or F radical which remains following removal of the two phenolic hydrogen atoms.

9. The particles as claimed in one of the preceding claims, where X is an epoxy group, isocyanate group, a group having at least one active hydrogen atom or a group having at least one ethylenically unsaturated double bond.

10. The particles as claimed in claim 9, where X is an epoxy group, an amino group or a group of the formula 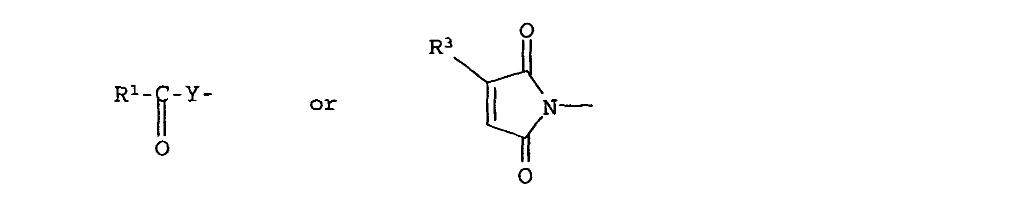 in which R¹ is the radical of an ethylenically unsaturated C₃-C₈ monocarboxylic acid or C₄-C₈ dicarboxylic acid that remains following removal of the carboxyl group and Y is O or NR², R² and R³ being H or C₁-C₄ alkyl.

11. The particles as claimed in claim 9 or 10, where R is -(CH₂)ₙ-,
- CH₂CHOHCH₂- or -CH₂CHOHCH₂-Z-, in which n is from 1 to 6 and Z is a bisphenol radical which remains following removal of the two phenolic hydrogen atoms.

12. The particles as claimed in claim 1, where
A is silicon dioxide, titanium dioxide or an Si/Al mixed oxide,
- B-X is (MeO)ₓMe(O)_{y1}(CH₂)ₙOCOCR⁴=CH₂ or
(MeO)ₓMe(O)_{y1}CH₂CHOHCH₂OCOCR⁴=CH₂, where Me is Si, Al, Ti or
Zr, x is 1 or 2, y1 is 0 or 1, n is from 2 to 6 and R⁴ is H or CH₃, the free valences of Si, Al, Ti or Zr being satisfied by alkoxy radicals and/or bonded to oxygen atoms of the same or a different core A.

13. The particles as claimed in claim 12, where MeOMe or MeOMeOMe has the definitions indicated in claim 7.

14. The particles as claimed in one of the preceding claims, where the free valences of Me are satisfied by alkyl, aryl, alkoxy, a group of the formula 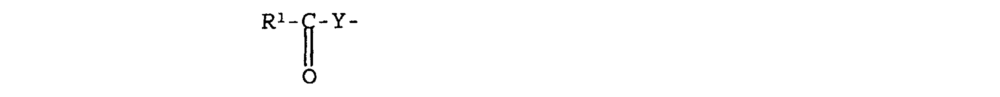 in which R¹ and Y have the definitions indicated in claim 10, or a group comprising phosphate.

15. The particles as claimed in claim 1, where the side chain is a polyalkyl hydrosiloxane radical.

16. The particles as claimed in one of the preceding claims having a glass transition temperature of the homopolymers of > 250°C.

17. A process for preparing the particles as claimed in one of claims 1 to 16, **characterized in that** the radicals B, -B-X and/or X are attached by covalent bonding to the core A, present in solid form, in the presence of strong acids.

18. The process s claimed in claim 17, **characterized in that** the covalent attachment is conducted in one process step (in situ).

19. A composition comprising the particles as claimed in one of claims 1 to 16.

20. The use of the particles as claimed in one of claims 1 to 16 as coating compositions, adhesive compositions, sealing compositions, molding compositions, casting compositions, polymer compositions or compositions for the dental, cosmetic or medical areas, lacquers, surface plating materials, or as binders for woodbase materials and stone compositions.

## Revendications

1. Particule d'oxyde métallique polymérisable résistant aux températures élevées, présentant une température de transition vitreuse de l'homopolymère ≥ 100°C et avec un noyau A constitué d'au moins un oxyde d'un métal ou d'un semi-métal du troisième au sixième groupe principal, du premier au huitième groupe secondaire du Système Périodique ou des lanthanides et avec au moins un groupe -(B)w-X lié par covalence au noyau par l'intermédiaire d'un ou de plusieurs atomes d'oxygène de l'oxyde, dans lequel w a pour valeur 0 ou 1 et B représente un reste répondant aux formules
- (MeO)x Me(O)y1 - (R)y2-ou -R(O)z-,
x ayant pour valeur 0 à 100, y1, y2 et z ayant pour valeur, indépendamment les uns des autres, 0 ou 1 et Me symbolisant un métal ou un semi-métal du troisième au sixième groupe principal ou du troisième au huitième groupe secondaire du Système Périodique, les valences libres de Me représentant une liaison à un autre atome d'oxygène du noyau A et/ou une liaison par l'intermédiaire d'un atome d'oxygène à un Me dans un autre groupe B et/ou une liaison à un atome d'oxygène d'un autre noyau A et/ou sont saturées par H, un reste organique et/ou un reste trialkylsilyloxy,
R représente un groupe alkyle, cycloalkyle, aryle, arylalkyle, alkylaryle, alcoxy, acyle, acyloxy divalent ou un reste qui subsiste après élimination de deux atomes d'hydrogène phénoliques d'un composé phénolique présentant au moins deux groupes hydroxyle phénoliques, R pouvant être, le cas échéant, substitué par un, deux ou trois restes, qui sont choisis indépendamment les uns des autres parmi hydroxy, alcoxy, halogène et, dans le cas de restes aryle ou cycloalkyle, également alkyle, et/ou pouvant être interrompus dans la chaîne par un ou deux atome(s) d'oxygène, et
X peut être lié à B par l'intermédiaire d'un chaînon Z, qui est choisi parmi O, NR², OCO, COO, NHCO ou CONH, R² représentant un atome d' hydrogène ou un groupe alkyle en C₁-C₄, et représente un groupe fonctionnel réactif ou un reste comportant un groupe fonctionnel réactif,
le groupe ou les groupes -(B)w-X constituant au moins 10 % en poids par rapport au poids total de la particule,
pouvant être obtenue par liaison covalente des restes B, B-X et/ou X au noyau A se présentant sous forme solide, en présence d'un acide fort.

2. Particule selon la revendication 1, le groupe ou les groupes -BX constituant au moins 20 % en poids par rapport au poids total de la particule.

3. Particule selon la revendication 1 ou 2, le noyau étant un oxyde de Si, Al, Ti ou Zr ou un mélange ou un oxyde mixte de ceux-ci.

4. Particule selon l'une quelconque des revendications précédentes, Me représentant un métal ou un semi-métal du troisième ou du quatrième groupe principal ou du quatrième groupe secondaire du Système Périodique.

5. Particule selon la revendication 4, Me représentant Si, Ti, Al ou Zr.

6. Particule selon l'une quelconque des revendications précédentes, B étant choisi parmi :
a) -Me (O)_{y1}-R-
b) -Me-O-Me (O)_{y1-}R-
c) -Me-O-Me-O-Me (O)_{y1}-R-
d) -R-O-
e) -R-
où y1 a pour valeur 0 ou 1 et R possède les significations indiquées dans la revendication 1.

7. Particule selon la revendication 6, -Me-O-Me-dans le reste B étant choisi parmi :
- Si-O-Si-
- Si-O-Al-
- Si-O-Ti-
- Si-O-Zr-
- Al-O-Ti-
- Al-O-Zr-
- Al-O-Al-
et -Me-O-Me-O-Me- dans le reste C représentant -Si-O-Ti-O-Zr-.

8. Particule selon l'une quelconque des revendications précédentes, R représentant un groupe alkyle, hydroxyalkyle, alcoxy ou acyloxy divalent ou un reste Bisphénol A, B ou F subsistant après élimination des deux atomes d'hydrogène phénoliques.

9. Particule selon l'une quelconque des revendications précédentes, x représentant un groupe époxy, un groupe isocyanate, un groupe comportant au moins un atome d'hydrogène actif ou un groupe comportant au moins une double liaison éthyléniquement insaturée.

10. Particule selon la revendication 9, x représentant un groupe époxy, un groupe amino ou un groupe répondant à la formule 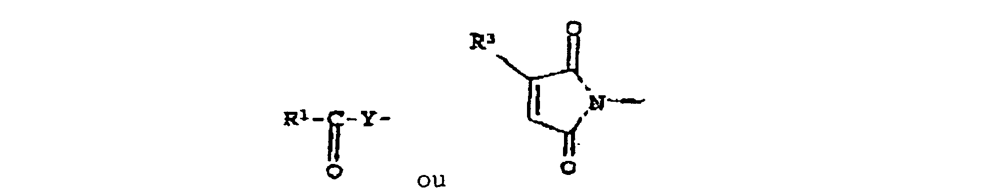 où R¹ symbolise le reste subsistant après élimination du groupe carboxyle d'un acide monocarboxylique en C₃-C₈ ou d'un acide dicarboxylique en C₄-C₈ éthyléniquement insaturé et Y représente O ou NR², R² et R³ représentant H ou un groupe alkyle en C₁-C_{4.}

11. Particule selon la revendication 9 ou 10, R représentant -(CH₂)ₙ-, -CH₂CHOHCH₂-, ou -CH₂CHOHCH₂- Z-, où n a pour valeur 1 à 6 et Z représente un reste Bisphénol subsistant après élimination des deux atomes d'hydrogène phénoliques.

12. Particule selon la revendication 1, dans laquelle
A représente le dioxyde de silicium, le dioxyde de titane, ou un oxyde mixte Si/Al,
- B-X représente (MeO)ₓMe(O)_{y1}(CH₂)ₙOCOCR⁴ = CH₂ ou (MeO)ₓMe(O)_{yl}CH₂CHOHCH₂OCOCR⁴ = CH₂,
Me représentant Si, Al, Ti, ou Zr, x ayant pour valeur 1 ou 2, y1 ayant pour valeur 0 ou 1, n ayant pour valeur 2 à 6 et R⁴ représentant H ou CH₂,
les valences libres de Si, Al, Ti ou Zr étant saturées par des restes alcoxy et/ou étant liées à des atomes d'oxygène du même noyau A ou d'un autre noyau A.

13. Particule selon la revendication 12, dans laquelle MeOMe et MeOMeOMe possèdent les significations indiquées dans la revendication 7.

14. Particule selon l'une quelconque des revendications précédentes, dans laquelle les valences libres de Me sont saturées par un groupe alkyle, aryle, alcoxy, un groupe répondant à la formule : 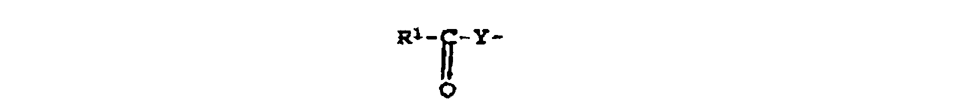 où R¹ et Y possèdent les significations indiquées dans la revendication 10, ou un groupe contenant un phosphate.

15. Particule selon la revendication 1, dans laquelle la chaîne latérale est un reste polyalkylhydrogénosiloxane.

16. Particule selon l'une quelconque des revendications précédentes, présentant une température de transition vitreuse de l'homopolymère >250°C.

17. Procédé de fabrication de la particule selon l'une quelconque des revendications 1 à 16, **caractérisé en ce qu'**on lie par covalence les restes B, -B-X et/ou X au noyau A se présentant sous forme solide en présence d'acides forts.

18. Procédé selon la revendication 17, **caractérisé en ce qu'**on effectue la liaison covalente au cours d'une étape de procédé (in situ).

19. Composition contenant la particule selon une quelconque des revendications 1 à 16.

20. Utilisation de la particule selon l'une quelconque des revendications 1 à 16 pour la fabrication de masses de revêtement, masses de couverture, masses adhésives, masses d'étanchéité, masses de moulage, masses de coulée, masses polymères ou masses pour les secteurs dentaire, cosmétique ou médical, peintures, produits d'amélioration des surfaces ou de liants pour matériaux à base de bois et masses de pierre.
